(19) Europäisches Patentamt European Patent Office Office européen des brevets

(11) **EP 3 977 969 B1**

(12) **EUROPEAN PATENT SPECIFICATION**

(45) Date of publication and mention
of the grant of the patent:
**03.12.2025 Bulletin 2025/49**

(21) Application number: **20815270.2**

(22) Date of filing: **25.05.2020**

(51) International Patent Classification (IPC):
*A61F 13/49* $^{(2006.01)}$     *A61F 13/494* $^{(2006.01)}$
*A61F 13/514* $^{(2006.01)}$     *A61F 13/515* $^{(2006.01)}$

(52) Cooperative Patent Classification (CPC):
**A61F 13/49017; A61F 13/4902**

(86) International application number:
**PCT/JP2020/020515**

(87) International publication number:
**WO 2020/241560 (03.12.2020 Gazette 2020/49)**

(54) **ABSORBENT ARTICLE**

ABSORBIERENDER ARTIKEL

ARTICLE ABSORBANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.05.2019 JP 2019102969**

(43) Date of publication of application:
**06.04.2022 Bulletin 2022/14**

(73) Proprietor: **Kao Corporation
Chuo-ku
Tokyo 103-8210 (JP)**

(72) Inventors:
• **SUZUKI, Takeshi
Haga-gun, Tochigi 321-3497 (JP)**
• **TOMITA, Mina
Haga-gun, Tochigi 321-3497 (JP)**

(74) Representative: **Vossius & Partner
Patentanwälte Rechtsanwälte mbB
Siebertstraße 3
81675 München (DE)**

(56) References cited:
JP-A- 2009 297 096    JP-A- 2009 297 096
JP-A- 2010 284 228    JP-A- 2012 087 452
JP-A- 2015 211 782    JP-A- 2017 176 882
JP-B1- 6 370 965

EP 3 977 969 B1

## Description

Technical Field

The present invention relates to an absorbent article.

Background Art

It has been studied in the field of absorbent articles such as disposable diapers to use a composite stretch sheet composed of at least two sheets having an elastic member fixed therebetween in a stretched state with a view to providing neat folds or corrugations. For example, the assignee common to this application proposed an absorbent article having folds created by a composite stretch sheet as disclosed in patent literatures 1 and 2 listed below. According to these literatures, the outer sheet that provides the exterior side of an article and an inner sheet laminated on the inner side of the outer sheet form respective folds such that the folds of the outer sheet are larger than those of the inner sheet. The difference in fold size between the inner and the outer sides of the composite stretch sheet creates a difference in cushioning properties between them.

Citation List

Patent Literature

Patent literature 1: JP 2009-297096A
Patent literature 2: JP 2013-176708A

Summary of Invention

The present invention relates to an absorbent article as defined in independent claim 1. Preferred embodiments are defined in the dependent claims.

Brief Description of Drawings

[Fig. 1] Fig. 1 is a perspective schematically illustrating an example of an elasticized leg region, in its streched state, of the absorbent article according to the present invention.
[Fig. 2] Fig. 2 is a cross-section of the composite elasticized region shown in Fig. 1, taken along line II-II.
[Fig. 3] Fig. 3 is a schematic perspective of an example of an elasticized leg region, in its relaxed state, of the absorbent article of the present invention.
[Fig. 4] Fig. 4 is a cross-section of the composite elasticized region illustrated in Fig. 3, taken along line IV-IV.

[Fig. 5] Fig. 5 is a plan schematically illustrating the skin facing surface side (interior side) of an embodiment of the absorbent article according to the present invention in its flat-out, uncontracted configuration.

Description of Embodiments

With a view to creating corrugations with soft touch or handle, it is common to use a sheet material having low bending stiffness as a composite stretch sheet. However, mere use of a sheet material having low bending stiffness results in non-uniform gathering or formation of irregular corrugations. In the manufacture of absorbent articles having corrugations on both the exterior and the interior surface, it is preferred to form small corrugations on the exterior side to provide an improved appearance and to make larger corrugations on the interior side to reduce pressure marks on the skin. Patent literatures 1 and 2 neither describe nor suggest a structure with small corrugations on the exterior side and large corrugations on the interior side.

In the light of the above circumstances, the present invention relates to an absorbent article having a corrugated structure that exhibits an enhanced aesthetic appearance and hardly leaves pressure marks on the skin.

The present invention will be described on the basis of its preferred embodiments with reference to the accompanying drawings. The absorbent article of the present invention generally has a front portion adapted to be worn about the front of a wearer, a rear portion adapted to be worn about the back of the wearer, and a crotch portion located intermediate between the front and rear portions. The absorbent article has an oblong shape with a longitudinal direction, which corresponds to the direction from the front portion to the crotch portion to the rear portion, and a lateral direction perpendicular to the longitudinal direction. The crotch portion has a target zone where bodily waste will be located. The target zone is usually located in or near the longitudinal middle portion of the absorbent article.

The absorbent article usually includes a topsheet disposed on the skin facing surface side, a backsheet on the non-skin facing surface side, and an absorbent member between the topsheet and the backsheet. The topsheet is preferably a liquid permeable sheet, such as nonwoven fabric or perforated film. The backsheet is preferably a sparingly liquid permeable sheet, such as sparingly liquid permeable film or a composite laminate composed of sparingly liquid permeable film laminated with a sheet having a good hand, such as nonwoven fabric.

The absorbent member includes an absorbent core. The absorbent core is formed, e.g., of an airlaid web of hydrophilic fibers, such as cellulosic fibers, including pulp; an airlaid mixture of hydrophilic fibers and an absorbent polymer; a deposited layer of an absorbent polymer; or a stacked sheet structure composed of two absorbent sheets and an absorbent polymer held therebetween. The absorbent core may be covered on at least its skin facing surface, i.e., may be covered on its entire surface inclusive of the skin facing surface and the non-skin facing surface, with a liquid permeable core wrap sheet. The core wrap sheet may be, for example, tissue made of hydrophilic fibers or liquid permeable nonwoven fabric.

As used herein, the term "skin facing surface" refers to the side of an absorbent article or a member constituting the absorbent article (e.g., an absorbent member) that faces the wearer's skin while worn, i.e., the side relatively closer to the wearer's skin. The term "non-skin facing surface" refers to the side facing away from the wearer's skin while worn, i.e., the side relatively farther from the wearer's skin.

The absorbent article may further include, in addition to the topsheet, backsheet, and absorbent member, a leak-proof barrier cuff extending in the longitudinal direction according to the intended use of the article. The barrier cuff is disposed in each longitudinally extending lateral side portions of the article on the skin facing surface side. The barrier cuff usually has a proximal edge fixed on the skin facing surface side of the absorbent article and a free edge. The barrier cuff is configured to upstand on the skin facing surface side of the article with the free edge up. The barrier cuff is made of a liquid resistant or water repellent and breathable sheet material. The barrier cuff may have an elastic member, such as a rubber thread, fixed in a stretched state along or near the free edge. While the absorbent article is worn, the elastic member contracts to make the barrier cuff upstand toward the wearer's body, whereby liquid waste discharged and running on the topsheet is effectively prevented from leaking laterally outward from the absorbent article.

The longitudinally extending lateral side edges of the absorbent article are inwardly convex in the crotch portion. The inwardly convex portion of each lateral side is formed of the two sheets making up the absorbent article with elastic members, such as rubber threads, disposed therebetween in their stretched state. While the absorbent article is worn, the elastic members contract to form an elasticized leg region that is adapted to be worn around the wearer's leg.

Depending on the intended use of the absorbent article, the absorbent article may further have an elastic member, such as a rubber thread, disposed in at least one of the opposed longitudinal ends thereof and extending along the lateral direction in its stretched state. While the article is worn, the elastic member contracts to form waist gathers.

Examples of the absorbent article having the aforementioned structure include disposable open style diapers, disposable pull-on diapers, incontinence pads, and sanitary napkins.

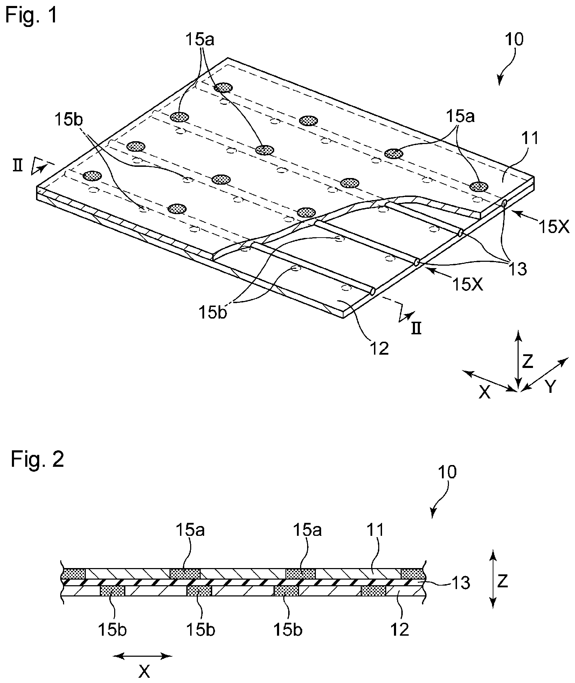

Figs. 1 and 2 illustrate an example of the elasticized leg region used in the absorbent article of the present invention. The elasticized leg region 10 depicted in Figs. 1 and 2 is in its uncontracted condition. The elasticized leg region 10 is provided in a leg portion, which is adapted to be worn around the wearer's leg, of such an absorbent article as the above-described disposable diapers, incontinence pad, or sanitary napkin.

As illustrated, the elasticized leg region 10 includes a first sheet 11, a second sheet 12, and elastic members 13 arranged in between the two sheets 11 and 12. The first sheet 11 is located closer to the wearer's skin than the elastic members 13, and the second sheet 12 is farther from the wearer's skin than the elastic members 13. The elastic members 13 each extend in the stretch direction X of the elasticized leg region 10 and are spaced in a direction intersecting the stretch direction X. In the embodiment illustrated in Fig. 2, the elastic members 13 are spaced in a perpendicular direction Y that is perpendicular to the stretch direction X. Each elastic member 13 preferably extends the whole length of the elasticized leg region 10. The elasticized leg region 10 is thus configured to stretch and contract in the stretch direction X, along which the elastic members extend. The elasticized leg region 10 is substantially inelastic in the perpendicular direction Y.

The first sheet 11 may be formed of any material usable to form the skin facing surface side surfaces of absorbent articles, including the sheet materials forming the topsheet or barrier cuffs and the sheet materials useful as the inner sheet making up an outer cover of absorbent articles. The second sheet 12 may be formed of any material usable to make the non-skin facing surface of absorbent articles, such as those used to form the backsheet or the outer sheet making up the outer cover. The two sheets 11 and 12 may be of the same or different materials.

The elastic members 13 are to take on the elastic contraction and extension functions of the elasticized leg region 10. Each elastic member 13 may have the shape of a thread (elastic filament), ribbon, film, or sheet composed of a fiber aggregate, preferably the shape of a thread or ribbon. The elastic members 13 shown in Fig. 1 are thread-like elements and are arranged at a substantially regular interval in the perpendicular direction Y. Otherwise, the elastic members 13 may be arranged at irregular intervals in the perpendicular direction Y.

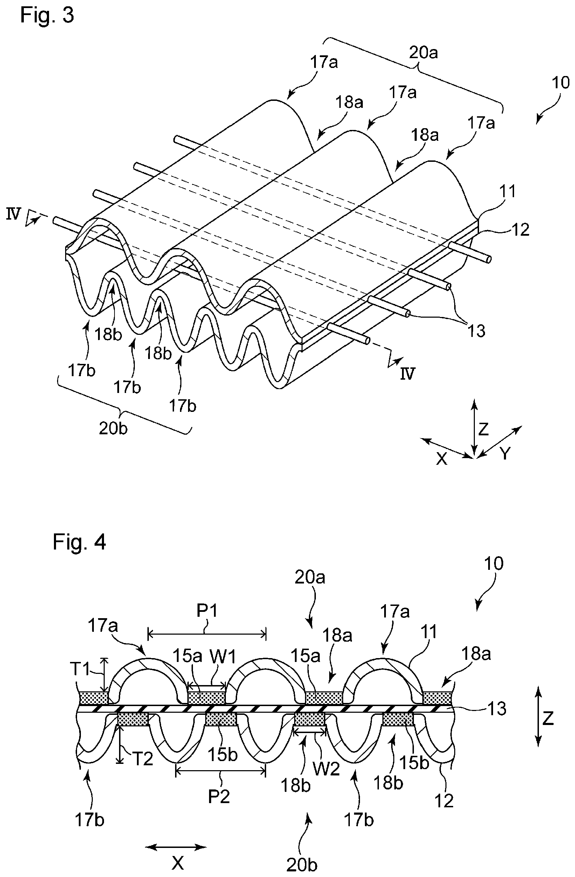

Figs. 3 and 4 display the elasticized leg region 10 in a contracted condition, i.e., in a relaxed state with no outer force applied. When the elasticized leg region 10 is in the contracted condition, the first sheet 11 and second sheet 12 are each deformed into ridges and troughs alternating in the stretch direction X. The first sheet 11 forms first ridges 17a that protrude outward away from the second sheet 12. The first ridges 17a extend in the perpendicular direction Y and are spaced from each other in the stretch direction X. Between the first ridges 17a adjacent in the stretch direction X is formed a first trough 18a extending in the perpendicular direction Y. Thus, the elasticized leg region 10 has, on its skin facing surface, a corrugated structure 20a composed of the first ridges 17a and the first troughs 18a resulting from deformation of the first sheet 11. The corrugations of the skin facing corrugated structure 20a extend in the perpendicular direction Y. Each first ridge 17a is preferably hollow from the viewpoint of softness of the corrugated structure.

Similarly to the first sheet 11, the second sheet 12 forms second ridges 17b protruding outward away from the first sheet 11 and second troughs 18b, all extending in the perpendicular direction Y. The second ridges 17b and second troughs 18b alternate in the stretch direction X. Thus, the elasticized leg region 10 has, on its non-skin facing surface, a corrugated structure 20b composed of the second ridges 17b and the second troughs 18b resulting from deformation of the second sheet 12. The corrugations of the non-skin facing corrugated structure 20b extend in the perpendicular direction Y. Similarly to the first ridges 17a, the second ridges 17b are preferably hollow from the viewpoint of softness of the corrugated structure.

Each of the elastic members 13 is bonded to the first sheet 11 at every first trough 18a with an adhesive. Each elastic member 13 is also bonded to the second sheet 12 at every second trough 18b with an adhesive. The first sheet 11 and the second sheet 12 are bonded to each other with an adhesive at the first troughs 18a and the second troughs 18b between elastic members 13 adjacent in the perpendicular direction Y. To achieve the bonding, any adhesive commonly used in the art, such as a hot-melt adhesive, may be used. The pattern of applying the adhesive will be described later.

As illustrated in Figs. 3 and 4, in a contracted condition of the elasticized leg region 10, the corrugations on the skin facing surface side of the corrugated structure 20a are larger than those on the non-skin facing surface side. In more detail, the first ridges 17a formed of the first sheet 11 have a pitch P1 that is larger than a pitch P2 of the second ridges 17b formed of the second sheet 12. As used herein, the term "pitch" refers to the repeat distance from one ridge crest to another adjacent in the stretch direction X. In the embodiment illustrated in Fig. 4, the locations of the first troughs 18a are not coincident with those of the second troughs 18b in the thickness direction Z, neither are the locations of the first ridges 17a and those of the second ridges 17b. Otherwise, in view of softness of the corrugated structure, a plurality of second ridges 17b may be located between two first ridges 17a adjacent in the stretch direction X, with one second trough 18b being located in the location of the first trough 18a, when viewed in the thickness direction Z.

The elasticized leg region 10 having the above-described structure has relatively small corrugations on the non-skin facing corrugated structure 20b and thereby exhibits an improved exterior appearance. In addition to this, since the corrugations of the skin facing corrugated structure 20a are relatively large, they are less likely to leave red marks on the skin. Having the thus configured elasticized leg regions 10, the absorbent article of the present invention exhibits improved esthetic design on not only the elasticized leg regions 10 per se but the entire exterior.

From the standpoint of appearance and prevention of leaving pressure marks, the ratio of pitch P1 to pitch P2 is preferably 1.2 or higher, more preferably 1.3 or higher, preferably 4.0 or lower, more preferably 3.8 or lower, specifically preferably 1.2 to 4.0, more preferably 1.3 to 3.8. To prevent leaving pressure marks, pitch P1 is preferably 3.5 mm or more, more preferably 3.8 mm or more, preferably 8.0 mm or less, more preferably 7.5 mm or less, specifically preferably 3.5 mm to 8.0 mm, more preferably 3.8 mm to 7.5 mm. In terms of appearance, pitch P2 is preferably 0.8 mm or more, more preferably 1.0 mm or more, preferably 3.0 mm or less, more preferably 2.8 mm or less, specifically preferably 0.8 mm to 3.0 mm, more preferably 1.0 mm to 2.8 mm.

In addition to the pitch P1 being greater than pitch P2, it is preferred that the first ridge 17a have a height T1 greater than a height T2 of the second ridge 17b. This configuration further improves the appearance and further prevents the corrugations from leaving pressure marks. From that viewpoint, the ratio of height T1 to height T2 is preferably 1.1 or higher, more preferably 1.2 or higher, preferably 3.0 or lower, more preferably 2.8 or lower, specifically preferably 1.1 to 3.0, more preferably 1.2 to 2.8. In view of reduction of pressure marks, the height T1 is preferably 0.4 mm or greater, more preferably 0.6 mm or greater, preferably 6.0 mm or smaller, more preferably 4.0 mm or smaller, specifically preferably 0.4 mm to 6.0 mm, more preferably 0.6 mm to 4.0 mm. In view of appearance, the height T2 is preferably 0.2 mm or greater, more preferably 0.5 mm or greater, preferably 5.0 mm or smaller, more preferably 3.0 mm or smaller, specifically preferably 0.2 mm to 5.0 mm, more preferably 0.5 mm to 3.0 mm.

With the view to providing a good appearance and reducing pressure marks, the first trough 18a preferably has a width W1 (see Fig. 4) in the stretch direction X of 0.2 mm or greater, more preferably 0.4 mm or greater, and preferably 5.0 mm or smaller, more preferably 3.0 mm or smaller, and the second trough 18b preferably has a width W2 (see Fig. 4) in the stretch direction X of 0.1 mm or greater, more preferably 0.2 mm or greater, and preferably 4.0 mm or smaller, more preferably 3.0 mm or smaller.

The pitches P1 and P2, heights T1 and T2, and widths W1 and W2 can be measured as follows. The elasticized leg region 10 in a contracted state is cut along the stretch direction X with a sharp razor, and the cut surface is observed in that state to measure the shortest distance between the crests of adjacent ridges on either side as the pitches P1 and P2 and the shortest distance between the upper plane of the crest of a ridge and the plane forming the bottom of a trough on either side as the height T1 and T2. Furthermore, the distances between high-density portions 15a adjacent in a direction perpendicular to the stretch direction X and the distances between high-density portions 15b adjacent in a direction perpendicular to the stretch direction X are measured on the cut surface, and the arithmetic mean of these distances are taken as the widths W1 and W2, respectively. The measurements are made by the naked eye. When unmeasurable by the naked eye, the cut surface may be observed using, for example, a microscope VHX-1000 from Keyence at a magnification of 20 to 100 times.

In order to form the first ridges 17a with pitch P1 greater than pitch P2 of the second ridges 17b, it is preferred for the first sheet 11 to have a lower ring crush compression strength than the second sheet 12. The ratio of the ring crush compression strength of the first sheet 11 to that of the second sheet 12 is preferably 0.20 or higher, more preferably 0.25 or higher, preferably 0.95 or lower, more preferably 0.90 or lower, specifically preferably 0.20 to 0.95, more preferably 0.25 to 0.90. The ring crush compression strength of the first sheet 11 is preferably 10.0 N or lower, more preferably 8.0 N or lower, even more preferably 7.5 N or lower. The lower limit is preferably 2.0 N. The ring crush compression strength of the second sheet 12 is preferably 16.0 N or lower, more preferably 15.0 N or lower, even more preferably 14.5 N or lower. The lower limit is 3.5 N. The ring crush compression strength is measured by the method below.

Method for measuring ring crush compression strength:

The elasticized leg region 10 is separated into the first sheet 11 and the second sheet 12 by peeling using a dryer, a cold spray, and the like. Otherwise, sheet materials to be used to form the first sheet 11 and the second sheet 12 may be provided. A rectangular piece measuring 150 mm in the stretch direction X which corresponds to the machine direction in the production of the sheet material and 30 mm in the perpendicular direction Y is cut out from each sheet. Both longitudinal ends of the cut piece are joined with a 5 mm wide overlap, and the overlap is secured with a staple (No. 10-1M, from Max Co., Ltd.) at each longitudinal end thereof to make a cylindrical specimen of 45 mm in diameter. Each staple is affixed in parallel with the circumferential direction. The cylindrical specimen is put upright on the mount of a compression tester (e.g., RTA-100, from Orientec Corp.) and axially compressed by a compression plate of at least 70 mm diameter at a rate of 10 mm/min. The maximum load applied (N/30 mm) during the compression is recorded. The measurement is taken on five specimens for each of the first sheet 11 and the second sheet 12, and the average of the measurement results is taken as a ring crush compression strength of the sheet.

With the view to adjusting the ring crush compression strength to a lower level and forming relatively small corrugations on the non-skin facing corrugated structure 20b, as illustrated in Figs. 1 and 2, the first sheet 11 and the second sheet 12 have a plurality of high-density portions 15b resulting from partial densification. When each of the first sheet 11 and the second sheet 12 has high-density portions, the area of the high-density portions 15b per unit area of the second sheet 12 is larger than the area of the high-density portions 15a per unit area of the first sheet 11. The high-density portion has a higher fiber density than the surroundings as a result of partial compaction and densification of the fibers making up the sheet. Having a higher fiber density, the high-density portion is stiffer than the surroundings. Such high-density portions can be formed by previously processing the sheet material by, for example, partial compression, such as embossing, or partial fiber entanglement by machine, such as needle punching or hydroentanglement. The plan-view shape of the high-density portions may be a circle or an anisotropic shape such as a rectangle or an ellipse.

The high-density portions are preferably spacedly arranged in the stretch direction X of the elasticized leg region 10. The individual high-density portions may be either continuous or discontinuous in the perpendicular direction Y. From the viewpoint of feel to the touch, the high-density portions are preferably arranged spacedly or discontinuously in both the stretch direction X and the perpendicular direction Y.

With the view to forming relatively small corrugations on the non-skin facing corrugated structure 20b, the ratio of the area of the high-density portions 15b per unit area of the second sheet 12 to that of the high-density portions 15a per unit area of the first sheet 11 is preferably 1.2 or higher, more preferably 1.3 or higher, preferably 4.0 or lower, more preferably 3.5 or lower, specifically preferably 1.2 to 4.0, more preferably 1.3 to 3.5. The area of the high-density portions 15a per unit area of the first sheet 11 is preferably 5.0% or larger, more preferably 5.5% or larger, and preferably 18.0% or smaller, more preferably 16.0% or smaller. The area of the high-density portions 15b per unit area of the second sheet 12 is preferably 8.0% or larger, more preferably 8.5% or larger, and preferably 20.0% or smaller, more preferably 19.0% or smaller. The individual high-density portions 15a preferably have an area in the plan view of 0.5 mm$^2$ or larger, more preferably 0.8 mm$^2$ or larger, and preferably 4.5 mm$^2$ or smaller, more preferably 4.0 mm$^2$ or smaller. The individual high-density portions 15b preferably have an area of 1.5 mm$^2$ or larger, more preferably 1.8 mm$^2$ or larger, and preferably 5.0 mm$^2$ or smaller, more preferably 4.5 mm$^2$ or smaller. The area of the high-density portions per unit area of the sheet can be measured as follows.

Method for measuring area of high-density portion per unit area of sheet:

The elastic members 13 are stretched out until the elasticized leg region 10 is straightened up to its design dimension. A rectangular piece measuring 100 mm in the stretch direction X and 15 mm in the perpendicular direction Y is cut out from the stretched elasticized leg region 10. The number of the high-density portions 15a of the first sheet 11 of the cut piece is counted, the area of the individual high-density portion 15a is measured, and the total area of the high-density portions 15a is calculated. The total area of the high-density portions 15a is divided by the area of the cut piece to give the area of the high-density portions 15a per unit area of the first sheet 11. The area of the high-density portions 15b of the second sheet 12 is obtained in the same manner as for the area of the high-density portions 15a.

As illustrated in Figs. 1 and 2, it is preferred for at least the second sheet 12, out of the first sheet 11 and the second sheet 12, of the elasticized leg region 10 to have a plurality of the high-density portions located at positions overlapping at least one elastic member 13, more preferably all the elastic members, 13 in the thickness direction Z. With the high-density portions being so arranged, it is easier to create a corrugated structure having the high-density portions neatly aligned in the bottom of the troughs along the perpendicular direction Y. In some detail, since the high-density portions have relatively high stiffness, the regions having the high-density portion remain adhesively bonded to the elastic members when the sheet is gathered by contraction of the elastic members to form the corrugated structure, thereby preventing unintended bulging of the sheet. The high-density portions located at the bottoms and their vicinities remain substantially flat. In contrast, the other regions of the sheet located between adjacent high-density portions are debonded on contraction of the elastic members and are easily allowed to bulge outward from the high-density portions in the thickness direction away from the elastic members. As a result, the ridges and troughs are easily formed with regularity to make a corrugated structure having an aesthetically pleasant appearance in a good alignment.

When the high-density portions 15a of the first sheet 11 and the high-density portions 15b of the second sheet 12 individually overlap the same elastic member 13 in the thickness direction Z as illustrated in Figs. 1 and 2, the number of the high-density portions 15b overlapping the elastic member 13 is preferably greater than that of the high-density portions 15a overlapping the elastic member 13. The above arrangement facilitates the formation of the corrugated structure 20a with the individual high-density portions 15a located at the bottom of the first troughs 18a on the skin facing surface side and the corrugated structure 20b with the individual high-density portions 15b located at the bottom of the second troughs 18b on the non-skin facing surface side. Additionally, the above arrangement allows for making the corrugations of the corrugated structure 20b on the non-skin facing surface side smaller than those of the corrugated structure 20a on the skin facing surface side as illustrated in Fig. 4.

To make the corrugations of the non-skin facing corrugated structure 20b relatively small, it is preferred that the ratio of the number of the high-density portions 15b overlapping one elastic member 13 to that of the high-density portions 15a overlapping the same elastic member 13 be 1.1 or higher, more preferably 1.2 or higher, preferably 3.0 or lower, more preferably 2.8 or lower, specifically preferably 1.1 to 3.0, more preferably 1.2 to 2.8. The number of the high-density portions 15a per 100 mm is preferably 2 or greater, more preferably 4 or greater, and preferably 20 or smaller, more preferably 16 or smaller. The number of the high-density portions 15b per 100 mm is preferably 4 or greater, more preferably 6 or greater, and preferably 30 or smaller, more preferably 26 or smaller.

Arranging the high-density portions 15a 15b in an overlapping relation with an elastic member 13 can preferably be achieved by, for example, providing sheet materials each having lines of high-density portions (lines 15X in Fig. 1) and stacking the sheet materials with elastic members 13 therebetween with the stretch direction X of each elastic member 13 coincident with the extending direction of each line 15X such that the individual lines 15X of each sheet material align with the individual elastic members 13 in the thickness direction. In that case, the interval of the elastic members 13 or the lines of the high-density portions can be adjusted as appropriate to the design dimensions of an intended absorbent article.

In order to adjust the ring crush compression strength to a lower level with the view to making the corrugations of the non-skin facing corrugated structure 20b relatively small, it is preferred for the second sheet 12 to have a greater work of compression (WC) than the first sheet 11. The work of compression is a measure of cushioning properties of sheet materials. A material having a greater WC value is evaluated as having higher cushioning properties. The ratio of the WC value of the second sheet 12 to that of the first sheet 11 is preferably 1.2 or higher, more preferably 1.4 or higher, preferably 5.0 or lower, more preferably 4.5 or lower, and specifically preferably 1.2 to 5.0, more preferably 1.4 to 4.5. The WC value of the first sheet 11 is preferably 0.015 gf·cm/cm$^2$ or greater, more preferably 0.018 gf·cm/cm$^2$ or greater, preferably 0.070 gf·cm/cm$^2$ or smaller, more preferably 0.065 gf·cm/cm$^2$ or smaller. The WC value of the second sheet 12 is preferably 0.018 gf·cm/cm$^2$ or greater, more preferably 0.025 gf·cm/cm$^2$ or greater, preferably 0.35 gf·cm/cm$^2$ or smaller, more preferably 0.32 gf·cm/cm$^2$ or smaller. The work of compression (WC) can be determined as follows using KESFB4-AUTO-A (trade name) from Kato Tech Co., Ltd. in accordance with the method described in Kawabata, Hideo, fuai hyouka no hyoujunka to kaiseki (Standardization and Analysis of Hand Evaluation), Japanese Hand Evaluation and Standardization Committee, The Textile Machinery Soc. of Japan, 2nd Ed. (Jul. 10, 1980).

Method for determining work of compression (WC):

The elasticized leg region 10 is separated into the first sheet 11 and the second sheet 12 by peeling using a dryer, a cold spray, and the like. Otherwise, sheet materials to be used to form the first sheet 11 and the second sheet 12 may be provided. A rectangular piece measuring 100 mm in the stretch direction X and 15 mm in the perpendicular direction Y is cut out from each sheet. Each cut piece is placed on the stage of a tester. The piece is then compressed by a circular flat steel plate with an area of 2 cm$^2$ at a rate of 0.02 cm/sec to a maximum load of 50 g/cm$^2$ and recovered at the same rate. The work of compression (WC) is represented by the formula below, where $T_m$ and $T_0$ represent the thickness under load of 49 cN/cm$^2$ and 0.49 cN/cm$^2$, respectively; and $P_a$ represents the load (cN/cm$^2$) applied in the compression phase.

[Math. 1]

$$WC = \int_{T_0}^{T_m} P_a dT$$

In order to adjust the ring crush compression strength to a lower level with the view to making the corrugations of the non-skin facing corrugated structure 20b relatively small, it is preferred for the second sheet 12 to have a larger thickness than the first sheet 11. The ratio of the thickness of the second sheet 12 to that of the first sheet 11 is preferably 2.0 or higher, more preferably 2.2 or higher, preferably 15.0 or lower, more preferably 14.5 or lower, and specifically preferably 2.0 to 15.0, more preferably 2.2 to 14.5. The thickness of the first sheet 11 is preferably 0.05 mm or larger, more preferably 0.06 mm or larger, and preferably 0.40 mm or smaller, more preferably 0.38 mm or smaller. The thickness of the second sheet 12 is preferably 0.10 mm or larger, more preferably 0.12 mm or larger, and preferably 1.20 mm or smaller, more preferably 1.15 mm or smaller.

The thickness of the sheets can be measured as follows. The elasticized leg region 10 is separated into the first sheet 11 and the second sheet 12 by peeling using a dryer, a cold spray, and the like. Otherwise, sheet materials to be used to form the first sheet 11 and the second sheet 12 may be provided. Each sheet is placed on a level surface without causing wrinkling, and its thickness with no load applied is measured using a digital microscope, e.g., VHX-1000 from Keyence.

In order to make it easier for the sheet to bulge outward in the thickness direction away from the elastic members between adjacent high-density portions, the first sheet 11 and the second sheet 12 are preferably bonded to each other between elastic members 13 adjacent in the perpendicular direction Y with an intermittently applied adhesive. The pattern of intermittent application of the adhesive may be, for example, a dot, helical, or stripe pattern (extending in parallel to the elastic members 13 and spaced in the perpendicular direction Y). The adhesive for bonding the elastic members 13 to the first sheet 11 or the second sheet 12 is preferably applied to the elastic members 13 in straight lines extending in the stretch direction X using, e.g., a comb gun.

In order to make it easier for the sheet to bulge outward in the thickness direction away from the elastic members between adjacent high-density portions, the bond strength between the first sheet 11 and the second sheet 12 between the elastic members 13 adjacent in the perpendicular direction Y is preferably 25.0 N/30 mm or less, more preferably 20.0 N/30 mm or less. The lower limit of that bond strength is preferably 1.0 N/30 mm. The bond strength is measured as follows.

Method for measuring bond strength:

The elastic members 13 are stretched out until the elasticized leg region 10 is straightened up to its design dimension. A rectangular piece measuring 50 mm in the stretch direction X and 30 mm in the perpendicular direction Y is cut out from the stretched elasticized leg region 10. The first sheet 11 and the second sheet 12 of the cut piece are peeled apart from one of the longitudinal ends, and the separated portions are clamped in the jaws of a tensile tester (e.g., Tensilon RTC series, from A and D Co., Ltd.) in a T-configuration at an initial jaw separation of 20 mm. The jaws are further separated at a rate of 300 mm/min to record the maximum strength. The measurement is made in triplicate, and the average maximum strength is taken as the bond strength in unit of N/30 mm.

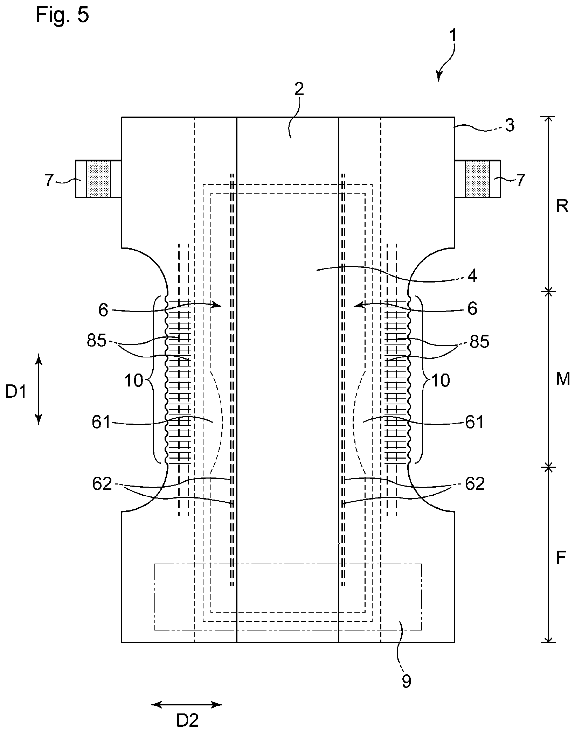

In Fig. 5 is illustrated an open style disposable diaper 1 as an embodiment of the absorbent article having the elasticized leg portion 10. The diaper 1 has, in its flat-out, uncontracted configuration, a longitudinal direction D1 corresponding to the front-to-back direction of a wearer, i.e., the direction from the front through the crotch to the back of a wearer and a lateral direction D2 that is perpendicular to the longitudinal direction D1. The diaper 1 includes a front portion F, a rear portion R, and a crotch portion M intermediate between the front and the rear portions. These three portions are continuous in the front-to-back direction of a wearer.

As used herein, the expression "flat-out, uncontracted configuration (of the diaper 1)" means a state in which the diaper 1 is in an open and flat condition with every elastic member stretched out until the diaper is straightened up to its design dimensions, i.e., with any influences of elastic members eliminated.

As illustrated in Fig. 5, the diaper 1 includes a liquid permeable topsheet 2 that forms the skin facing surface and a sparingly liquid permeable, inclusive of "water-repellant", backsheet 3 that forms the non-skin facing surface. In between the topsheet 2 and the backsheet 3 is interposed a generally rectangular absorbent member 4 with its longitudinal direction coincident with the longitudinal direction D1. The absorbent member 4 straddles the front portion F and the rear portion R. Both the topsheet 2 and the backsheet 3 are larger in size than the absorbent member 4, extending outward beyond the periphery of the absorbent member 4. The topsheet 2 and the backsheet 3 each extend laterally outward from each longitudinally extending, lateral side edge of the absorbent member 4. Each longitudinal end of the topsheet 2 and the backsheet 3 is substantially even with each longitudinal end of the diaper 1.

As illustrated in Fig. 5, the diaper 1 includes a pair of leak barrier cuffs 6 provided on the skin facing surface in the laterally opposite side edge portions to extend in the longitudinal direction D1. The barrier cuff 6 is formed of a water-repellant and air-permeable cuff-forming sheet 61. Each barrier cuff 6 has, along its free edge, at least one thread-like elastic member 62 provided in its stretched state in the longitudinal direction D1. The barrier cuff 6 upstands in at least the crotch portion M by the contraction of the elastic member 62 during wear, thereby to prevent lateral leakage of bodily waste such as urine.

As illustrated in Fig. 5, the cuff-forming sheet 61 forms a side flap together with the backsheet 3 extending laterally outward from the absorbent member 4. The side flap is formed of the part of the two sheets, i.e., the cuff-forming sheet 61 and the backsheet 3, that extends laterally outward from the absorbent member 4. Each of the side flaps extending in the longitudinal direction D1 in the lateral side portions of the diaper 1 has a plurality of leg elastic members 85 in their stretched state disposed between the two sheets to form leg gathers. Contraction of the leg elastic members 85 provides the elasticized leg region 10.

In the diaper 1 of Fig. 5, the stretch direction X of the elasticized leg region 10 is coincident with longitudinal direction D1 of the diaper, and the perpendicular direction Y of the elasticized leg region 10 is coincident with the lateral direction D2 of the diaper 1. In the elasticized leg region 10 of the diaper 1, the leg elastic members 85 correspond to the elastic members 13, the cuff-forming sheet 61 to the first sheet 11, and the backsheet 3 to the second sheet 12. When the elasticized leg region 10 of the diaper 1 is in a contracted condition, the cuff-forming sheet 61 and the backsheet 3 each gather to form ridges and troughs alternately in the stretch direction X such that the corrugations formed of the cuff-forming sheet 61 are larger than those formed of the backsheet 3. So configured, the elasticized leg region 10 hardly leaves pressure marks from the corrugated structure of the cuff-forming sheet 61 on the wearer's skin. In addition, the relatively small corrugations of the backsheet 3 provide an enhanced aesthetic appearance. From the viewpoint of aesthetics, the backsheet 3 is preferably formed of a composite laminate composed of sparingly water permeable film and nonwoven fabric on the non-skin facing surface of the film.

As illustrated in Fig. 5, the diaper 1 includes a fastening tape 7 provided along each lateral side edge of the rear portion R. The fastening tape 7 includes an attachment portion formed of a male component of a mechanical fastener. The diaper 1 has, on its non-skin facing surface of the front portion F, a landing zone 9 to which the attachment portion of the fastening tape 7 is releasably attached. The landing zone 9 is formed by fixing a female component of a mechanical fastener to the non-skin facing surface of the backsheet 3 in the front portion F by a known bonding method, such as adhesive bonding or heat sealing.

While the illustrated diaper 1 does not have elastic members in the longitudinal end portions of the front portion F and the rear portion R, i.e., a waist portion, elastic members may be disposed in their stretched state along the lateral direction D1 between the two sheets making up the waist portion, thereby to elasticize the waist portion.

With a view to enhancing the flexibility and hand of the elasticized leg region 10, the elasticized leg region 10 preferably has an overall basis weight of 10 $g/cm^2$ or more, more preferably 12 $g/cm^2$ or more, even more preferably 15 $g/cm^2$ or more, and preferably 40 $g/cm^2$ or less, more preferably 36 $g/cm^2$ or less, even more preferably 32 $g/cm^2$ or less.

The sheets 11 and 12 forming the elasticized leg region 10 may be any of various types of fibrous sheets, including nonwoven fabrics. Examples of useful nonwoven fabrics include spun-bonded, thermally bonded (such as point bonded, heat embossed, or through-air), hydroentangled, melt-blown, air-laid, resin-bonded, needle-punched, and wet-laid nonwoven fabrics. The sheet material may have a single layer or multilayer structure or a laminate structure composed of nonwoven fabric laminated with resin film.

In using nonwoven fabric as the sheets 11 and 12, the constituent fibers may be made from various thermoplastic resins, including polyolefins (such as polyethylene, polypropylene, and polybutene), polyesters (such as polyethylene terephthalate and polybutylene terephthalate), polyamides (such as nylon), vinyl resins (such as polystyrene and polyvinyl chloride), acrylonitrile resins (such as acrylic resins), methacrylic resins, and vinylidene resins; natural fibers, such as cotton and pulp; biodegradable plastics, such as polylactic acid; or polyblends composed of two or more of the above-enumerated resins. Conjugate fibers composed of two or more of the resins, such as sheath/core fibers, side-by-side fibers, self-crimping eccentric sheath/core fibers, and splittable fibers, are also useful. The fibers may have added thereto small amounts of additives, such as colorants, antistatics, lubricants, and hydrophilizing agents.

The elastic member 13 constituting the elasticized leg region 10 may be made of, e.g., elastic resins, including synthetic rubbers, such as styrene-butadiene rubber, butadiene rubber, isoprene rubber, and neoprene rubber, natural rubber, EVA rubber, styrene-isoprene-styrene (SIS) rubber, styrene-ethylene-butylene-styrene (SEBS) rubber, styrene-ethylene-propylene-styrene (SEPS) rubber, stretch polyolefins, such as ethylene-based elastomers and propylene-based elastomers, and polyurethanes. Exemplary forms of the elastic members include sheets, such as films and nets; threads or strings (or flat rubbers) with a rectangular, square, circular or polygonal section; and multifilamentous twine.

The number of the elastic members to be disposed in the elasticized leg region 10 is preferably 1 or greater, more preferably greater than 1, and preferably smaller than 7, more preferably smaller than 5. When an imaginary straight line perpendicular to the direction of extension of the elastic members is drawn, the number of the elastic members intersecting the imaginary line is defined to be the number of the elastic members disposed in the elasticized leg region 10. When there are two or more elasticized leg regions 10 in an absorbent article, the above-recited preferred range of the number of the elastic members is independently applied to each elasticized leg region.

The number of the elastic members 13 each overlapping the line 15X of high-density portions 15a of the first sheet 11 is preferably at least 1, more preferably greater than 1, and preferably smaller than 7, more preferably smaller than 5. The number of the elastic members 13 each overlapping the line 15X of high-density portions 15b of the second sheet 12 is preferably at least 1, more preferably greater than 1, and preferably smaller than 7, more preferably smaller than 5. Such arrangement provides a corrugated structure exhibiting neat alignment and thereby an enhanced aesthetic appearance.

While the absorbent article of the present invention has been described with particular reference to an embodiment in which the elasticized leg region 10 is used in an open style disposable diaper, the type of the absorbent article according to the present invention is not particularly limited. For instance, the present invention may be applied to disposable pull-on diapers of which the outer cover may have the elasticized leg regions 10 as a pair of gathered leg flaps. The invention may also be applied to panty type sanitary napkins having the elasticized leg regions 10 as gathered leg cuffs.

While in the embodiment shown in Fig. 5 the stretch direction X of the elasticized leg region 10 is coincident with the longitudinal direction D1 of the diaper 1, and the leg elastic members 85 are arranged in parallel to the longitudinal direction D1, the leg elastic members may be disposed along the curved leg opening receiving the wearer's leg.

Industrial Applicability

The absorbent article of the present invention exhibits an enhanced aesthetic appearance and hardly leaves pressure marks on the skin.

**Claims**

1. An absorbent article comprising a front portion (F) adapted to be worn about the front of a wearer, a rear portion (R) adapted to be worn about the back of a wearer, and a crotch portion (M) intermediate between the front and the rear portion,

   the absorbent article having, in the crotch portion (M), an elasticized leg region (10) adapted to be located around a leg of a wearer,
   the elasticized leg region (10) having a stretch direction (X) and comprising a first sheet (11) on the skin facing surface side, a second sheet (12) on the non-skin facing surface side, and one or more elastic members (13) between the first and second sheets, the stretch direction (X) of the elasticized leg region (10) being coincident with longitudinal direction (D1) of the absorbent article,
   the one or more elastic members (13) extending in the stretch direction (X) and being spacedly arranged in a direction intersecting the stretch direction (X) and bonded to the first and second sheets with an adhesive,
   the first sheet (11) having a lower ring crush compression strength than the second sheet (12), wherein the ring crush compression strength is measured with the method as defined in paragraph [0036] of the A1-publication,
   the elasticized leg region (10) being configured to

      deform the first sheet (11) to form a skin facing corrugated structure having first ridges (17a) and first troughs (18a) in a contracted state, the first trough (18a) being located between the first ridges (17a), and deform the second sheet (12) to form a non-skin facing corrugated structure having second ridges (17b) and second troughs, the second trough (18b) being located between the second ridges (17b), and

   the first ridges (17a) being arrayed at a longer pitch than the second ridges (17b),
   wherein the first and the second sheet each have one or more high-density portions (15a, 15b) resulting from partial densification of the sheets,
   the second sheet (12) having a larger area of the high-density portion(s) per unit area than the first sheet (11),
   wherein each of the elastic members (13) is bonded to the first sheet (11) at every first trough (18a) with an adhesive, and each elastic member (13) is also bonded to the second sheet (12) at every second trough (18b) with an adhesive, wherein the first sheet (11) and the second sheet (12) are bonded to each other with an adhesive at the first troughs (18a) and the second troughs (18b) between elastic members (13) adjacent in the perpendicular direction (Y).

2. The absorbent article according to claim 1, wherein a ratio of the pitch of the first ridges (17a) to the pitch of the second ridges (17b) is 1.2 to 4.0.

3. The absorbent article according to claim 1 or 2, wherein the first ridges (17a) have a larger height than the second ridges (17b).

4. The absorbent article according to any one of claims 1 to 3, wherein a ratio of the height of the first ridges (17a) to the height of the second ridges (17b) is 1.1 to 3.0.

5. The absorbent article according to any one of claims 1 to 4, wherein a ratio of the ring crush compression strength of the first sheet (11) to the ring crush compression strength of the second sheet (12) is 0.20 to 0.95.

6. The absorbent article according to any one of claims 1 to 5, wherein the first and the second sheet (10, 11) each have one or more high-density portions resulting from partial densification of the sheets at one or more positions overlapping the elastic member(s), and the second sheet (12) has a greater number of the high-density portions (15b) located at positions overlapping the elastic member(s) than the first sheet (11).

7. The absorbent article according to any one of claims 1 to 6, wherein the first and the second sheet each have one or more high-density portions resulting from partial densification of the sheet, each high-density portion (15a) of the first sheet (11) being at the bottom the first trough (18a), and each high-density portion (15b) of the second sheet (12) being at the bottom of the second trough (18b).

8. The absorbent article according to any one of claims 1 to 7, wherein a ratio of the area of the one or more high-density portions per unit area of the second sheet (12) to that of the first sheet (11) is 1.2 to 4.0.

9. The absorbent article according to any one of claims 1 to 8, wherein a ratio of the number of the one or more high-density portions (15b) of the second sheet (12) overlapping the individual elastic member to the number of the one or more high-density portions (15a) of the first sheet (11) overlapping the individual elastic member is 1.1 to 3.0.

10. The absorbent article according to any one of claims 1 to 9, wherein the second sheet (12) has a greater work of compression than the first sheet (11).

11. The absorbent article according to claim 10, wherein a ratio of the work of compression of the second sheet (12) to that of the first sheet (11) is 1.2 to 5.0.

12. The absorbent article according to any one of claims 1 to 11, wherein the second sheet (12) is thicker than the first sheet (11).

13. The absorbent article according to claim 12, wherein a ratio of the thickness of the second sheet (12) to the thickness of the first sheet (11) is 2.0 to 15.0.

14. The absorbent article according to any one of claims 1 to 13, wherein the second sheet (12) is formed of a composite laminate composed of sparingly water permeable film and nonwoven fabric on the non-skin facing surface side of the film.

**Patentansprüche**

1. Absorbierender Artikel, der einen vorderen Abschnitt (F), der angepasst ist, um um die Vorderseite eines Trägers herum getragen zu werden, einen hinteren Abschnitt (R), der angepasst ist, um um die Rückseite eines Trägers herum getragen zu werden, und einen Schrittabschnitt (M) aufweist, der zwischen dem vorderen und dem hinteren Abschnitt liegt,

wobei der absorbierende Artikel in dem Schrittabschnitt (M) einen elastifizierten Beinbereich (10) aufweist, der angepasst ist, um sich um ein Bein eines Trägers herum zu befinden, wobei der elastifizierte Beinbereich (10) eine Dehnungsrichtung (X) aufweist und eine erste Lage (11) auf der der Haut zugewandten Oberflächenseite, eine zweite Lage (12) auf der nicht der Haut zugewandten Oberflächenseite und ein oder mehrere elastische Elemente (13) zwischen der ersten und der zweiten Lage aufweist, wobei die Dehnungsrichtung (X) des elastifizierten Beinbereichs (10) mit einer Längsrichtung (D1) des absorbierenden Artikels übereinstimmt, wobei sich das eine oder die mehreren elastischen Elemente (13) in der Dehnungsrichtung (X) erstrecken und in einer Richtung, die die Dehnungsrichtung (X) schneidet, beabstandet eingerichtet sind und mit einem Klebstoff an die erste und die zweite Lage gebunden sind, wobei die erste Lage (11) eine geringere Ringstauchdruckfestigkeit als die zweite Lage (12) aufweist, wobei die Ringstauchdruckfestigkeit mit dem Verfahren wie in Absatz [0036] der A1-Veröffentlichung definiert gemessen wird, wobei der elastifizierte Beinbereich (10) konfiguriert ist zum

Verformen der ersten Lage (11), um eine der Haut zugewandte gewellte Struktur auszubilden, die erste Rippen (17a) und erste Vertiefungen (18a) in einem kontrahierten Zustand aufweist, wobei sich die erste Vertiefung (18a) zwischen den ersten Rippen (17a) befindet, und

Verformen der zweiten Lage (12), um eine nicht der Haut zugewandte gewellte Struktur auszubilden, die zweite Rippen (17b) und zweite Vertiefungen aufweist, wobei sich die zweite Vertiefung (18b) zwischen den zweiten Rippen (17b) befindet, und

wobei die ersten Rippen (17a) in einem größeren Abstand als die zweiten Rippen (17b) angeordnet sind,

wobei die erste und die zweite Lage jeweils einen oder mehrere hochverdichtete Abschnitte (15a, 15b) aufweisen, die aus einer teilweisen Verdichtung der Lagen resultieren,

wobei die zweite Lage (12) pro Flächeneinheit eine größere Fläche des/der hochverdichteten Abschnitts/Abschnitte als die erste Lage (11) aufweist,

wobei jedes der elastischen Elemente (13) an jeder ersten Vertiefung (18a) mit einem Klebstoff mit der ersten Lage (11) gebunden ist und jedes elastische Element (13) ebenso an jeder zweiten Vertiefung (18b) mit einem Klebstoff mit der zweiten Lage (12) gebunden ist, wobei die erste Lage (11) und die zweite Lage (12) an den ersten Vertiefungen (18a) und den zweiten Vertiefungen (18b) zwischen elastischen Elementen (13), die in der senkrechten Richtung (Y) angrenzen, mit einem Klebstoff miteinander gebunden sind.

2. Absorbierender Artikel nach Anspruch 1, wobei das Verhältnis des Abstands der ersten Rippen (17a) zu dem Abstand der zweiten Rippen (17b) 1,2 bis 4,0 beträgt.

3. Absorbierender Artikel nach Anspruch 1 oder 2, wobei die ersten Rippen (17a) eine größere Höhe als die zweiten Rippen (17b) aufweisen.

4. Absorbierender Artikel nach einem der Ansprüche 1 bis 3, wobei ein Verhältnis der Höhe der ersten Rippen (17a) zu der Höhe der zweiten Rippen (17b) 1,1 bis 3,0 beträgt.

5. Absorbierender Artikel nach einem der Ansprüche 1 bis 4, wobei ein Verhältnis der Ringstauchdruckfestigkeit der ersten Lage (11) zu der Ringstauchdruckfestigkeit der zweiten Lage (12) 0,20 bis 0,95 beträgt.

6. Absorbierender Artikel nach einem der Ansprüche 1 bis 5, wobei die erste und die zweite Lage (10, 11) jeweils einen oder mehrere hochverdichtete Abschnitte aufweisen, die aus der teilweisen Verdichtung der Lagen an einer oder mehreren Positionen resultieren, die das/die elastische(n) Element(e) überlappen, und die zweite Lage (12) eine größere Anzahl von hochverdichteten Abschnitten (15b), die sich an Positionen befinden, die das/die elastische(n) Element(e) überlappen, als die erste Lage (11) aufweist.

7. Absorbierender Artikel nach einem der Ansprüche 1 bis 6, wobei die erste und die zweite Lage jeweils einen oder mehrere hochverdichtete Abschnitte aufweisen, die aus der teilweisen Verdichtung der Lage resultieren, wobei sich jeder hochverdichtete Abschnitt (15a) der ersten Lage (11) an dem Boden der ersten Vertiefung (18a) befindet und sich jeder hochverdichtete Abschnitt (15b) der zweiten Lage (12) an dem Boden der zweiten Vertiefung (18b) befindet.

8. Absorbierender Artikel nach einem der Ansprüche 1 bis 7, wobei ein Verhältnis der Fläche des einen oder der mehreren hochverdichteten Abschnitte pro Flächeneinheit der zweiten Lage (12) zu der der ersten Lage (11) 1,2 bis 4,0 beträgt.

9. Absorbierender Artikel nach einem der Ansprüche 1 bis 8, wobei ein Verhältnis der Anzahl des einen oder der mehreren hochverdichteten Abschnitte (15b) der zweiten Lage (12), die das einzelne elastische Element überlappen, zu der Anzahl des einen oder der mehreren hochverdichteten Abschnitte (15a) der ersten Lage (11), die das einzelne elastische Element überlappen, 1,1 bis 3,0 beträgt.

10. Absorbierender Artikel nach einem der Ansprüche 1 bis 9, wobei die zweite Lage (12) eine größere Kompressionsarbeit als die erste Lage (11) aufweist.

11. Absorbierender Artikel nach Anspruch 10, wobei ein Verhältnis der Kompressionsarbeit der zweiten Lage (12) zu der der ersten Lage (11) 1,2 bis 5,0 beträgt.

12. Absorbierender Artikel nach einem der Ansprüche 1 bis 11, wobei die zweite Lage (12) dicker als die erste Lage (11) ist.

13. Absorbierender Artikel nach Anspruch 12, wobei ein Verhältnis der Dicke der zweiten Lage (12) zu der Dicke der ersten Lage (11) 2,0 bis 15,0 beträgt.

14. Absorbierender Artikel nach einem der Ansprüche 1 bis 13, wobei die zweite Lage (12) aus einem Verbundlaminat ausgebildet ist, das aus einer kaum wasserdurchlässigen Folie und einem Vliesstoff auf der nicht der Haut zugewandten Oberflächenseite der Folie besteht.

**Revendications**

1. Article absorbant comprenant une partie avant (F) adaptée pour être portée autour de l'avant d'un utilisateur, une partie arrière (R) adaptée pour être portée autour du dos d'un utilisateur, et une partie d'entrejambe (M) intermédiaire entre la partie avant et la partie arrière,

   l'article absorbant ayant, dans la partie d'entrejambe (M), une région de jambe élastique (10) adaptée pour être placée autour d'une jambe d'un utilisateur,
   la région de jambe élastique (10) ayant une direction d'étirement (X) et comprenant une première feuille (11) sur le côté de la surface tournée vers la peau, une seconde feuille (12) sur le côté de la surface non tournée vers la peau, et un ou plusieurs éléments élastiques (13) entre les première et seconde feuilles, la direction d'étirement (X) de la région de jambe élastique (10) coïncidant avec la direction longitudinale (D1) de l'article absorbant,
   le ou les éléments élastiques (13) s'étendant dans la direction d'étirement (X) et étant agencés de manière espacée dans une direction croisant la direction d'étirement (X) et liés aux première et seconde feuilles avec un adhésif,
   la première feuille (11) ayant une résistance à la compression par écrasement à l'anneau inférieure à celle de la seconde feuille (12), dans lequel la résistance à la compression par écrasement à l'anneau est mesurée avec le procédé tel que défini au paragraphe [0036] de la publication A1,
   la région de jambe élastique (10) étant conçue pour
   déformer la première feuille (11) pour former une structure ondulée tournée vers la peau ayant des premières crêtes (17a) et des premiers creux (18a) dans un état contracté, le premier creux (18a) étant situé entre les premières crêtes (17a), et
   déformer la seconde feuille (12) pour former une structure ondulée non tournée vers la peau ayant des secondes crêtes (17b) et des seconds creux, le second creux (18b) étant situé entre les secondes crêtes (17b), et
   les premières crêtes (17a) étant disposées selon un pas plus long que les secondes crêtes (17b),
   dans lequel la première et la seconde feuille ont chacune une ou plusieurs parties à haute densité (15a, 15b) résultant d'une densification partielle des feuilles,
   la seconde feuille (12) ayant une plus grande surface de la ou des parties à haute densité par unité de surface que la première feuille (11),
   dans lequel chacun des éléments élastiques (13) est lié à la première feuille (11) au niveau de chaque premier creux (18a) avec un adhésif, et chaque élément élastique (13) est également lié à la seconde feuille (12) au niveau de chaque second creux (18b) avec un adhésif, dans lequel la première feuille (11) et la seconde feuille (12) sont liées l'une à l'autre avec un adhésif au niveau des premiers creux (18a) et des seconds creux (18b) entre des éléments élastiques (13) adjacents dans la direction perpendiculaire (Y).

2. Article absorbant selon la revendication 1, dans lequel un rapport entre le pas des premières crêtes (17a) et le pas des secondes crêtes (17b) va de 1,2 à 4,0.

3. Article absorbant selon la revendication 1 ou 2, dans lequel les premières crêtes (17a) ont une hauteur plus importante que les secondes crêtes (17b).

4. Article absorbant selon l'une quelconque des revendications 1 à 3, dans lequel un rapport entre la hauteur des premières crêtes (17a) et la hauteur des secondes crêtes (17b) va de 1,1 à 3,0.

5. Article absorbant selon l'une quelconque des revendications 1 à 4, dans lequel un rapport entre la résistance à la compression par écrasement à l'anneau de la première feuille (11) et la résistance à la compression par écrasement à l'anneau de la seconde feuille (12) va de 0,20 à 0,95.

6. Article absorbant selon l'une quelconque des revendications 1 à 5, dans lequel la première et la seconde feuille (10, 11) ont chacune une ou plusieurs parties à haute densité résultant d'une densification partielle des feuilles à une ou plusieurs positions chevauchant le ou les éléments élastiques, et la seconde feuille (12) a un plus grand nombre de parties à haute densité (15b) situées à des positions chevauchant le ou les éléments élastiques que la première feuille (11).

7. Article absorbant selon l'une quelconque des revendications 1 à 6, dans lequel la première et la seconde feuille ont chacune une ou plusieurs parties à haute densité résultant d'une densification partielle de la feuille, chaque partie à haute densité (15a) de la première feuille (11) étant au fond du premier creux (18a), et chaque partie à haute densité (15b) de la seconde feuille (12) étant au fond du second creux (18b).

**8.** Article absorbant selon l'une quelconque des revendications 1 à 7, dans lequel un rapport entre la surface de la ou des parties à haute densité par unité de surface de la seconde feuille (12) et celle de la première feuille (11) va de 1,2 à 4,0.

**9.** Article absorbant selon l'une quelconque des revendications 1 à 8, dans lequel un rapport entre le nombre de la ou des parties à haute densité (15b) de la seconde feuille (12) chevauchant l'élément élastique individuel et le nombre de la ou des parties à haute densité (15a) de la première feuille (11) chevauchant l'élément élastique individuel va de 1,1 à 3,0.

**10.** Article absorbant selon l'une quelconque des revendications 1 à 9, dans lequel la seconde feuille (12) a un travail de compression supérieur à celui de la première feuille (11).

**11.** Article absorbant selon la revendication 10, dans lequel un rapport entre le travail de compression de la seconde feuille (12) et celui de la première feuille (11) va de 1,2 à 5,0.

**12.** Article absorbant selon l'une quelconque des revendications 1 à 11, dans lequel la seconde feuille (12) est plus épaisse que la première feuille (11).

**13.** Article absorbant selon la revendication 12, dans lequel un rapport entre l'épaisseur de la seconde feuille (12) et l'épaisseur de la première feuille (11) va de 2,0 à 15,0.

**14.** Article absorbant selon l'une quelconque des revendications 1 à 13, dans lequel la seconde feuille (12) est formée d'un stratifié composite composé d'un film faiblement perméable à l'eau et d'un non-tissé sur le côté de la surface non tournée vers la peau du film.

Fig. 1

Fig. 2

Fig. 3

Fig. 4

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2009297096 A

- JP 2013176708 A

### Non-patent literature cited in the description

- Standardization and Analysis of Hand Evaluation. **KAWABATA, HIDEO**. Japanese Hand Evaluation and Standardization Committee, The Textile Machinery Soc. of Japan. 10 July 1980